# EUROPÄISCHE PATENTSCHRIFT

(11) **EP 1 584 617 B1**
(45) Veröffentlichungstag und Bekanntmachung des Hinweises auf die Patenterteilung: **03.02.2010**
(21) Anmeldenummer: 04008314.9
(22) Anmeldetag: 06.04.2004
(51) Int. Cl.: C07D 213/20, C09K 19/34, B01J 31/00

(54) **Alkylpyridiniumdicyanamide als polare Lösungsmittel**
Alkylpyridiniumdicyanamides as polar solvents
Alkylpyridiniumdicyanamides comme solvants polaires

(43) Veröffentlichungstag der Anmeldung: 12.10.2005
(73) Patentinhaber: Lonza AG, 4052 Basel (CH)
(72) Erfinder: Taeschler, Christoph, 3912 Termen (CH)

(56) Entgegenhaltungen:
- OHLIN, C. ANDRE ET AL: "Carbon monoxide solubility in ionic liquids: determination, prediction and relevance to hydroformylation" CHEMICAL COMMUNICATIONS, CODEN: CHCOFS; ISSN: 1359-7345, 1. April 2004 (2004-04-01), Seiten 1070-1071, XP002294437 CAMBRIDGE UK
- D.R. MACFARLANE ET AL.: "Low viscosity ionic liquids based on organic salts of the dicyanamide anion" CHEMICAL COMMUNICATIONS, CODEN: CHCOFS; ISSN: 1359-7345, 2001, Seiten 1430-1431, XP002294438 CAMBRIDGE UK
- S.A. FORSYTH ET AL.: "Rapid, clean, and mild O-acetylation of alcohols and carbohydrates in an ionic liquid" CHEMICAL COMMUNICATIONS, CODEN CHCOFS, ISSN 1359-7345, 2002, Seiten 714-715, XP002294439 GB ROYAL SOCIETY OF CHEMISTRY.
- A. BÖSSMANN ET AL.: "Activation, tuning and immobilization of homogeneous catalysts in an ionic liquid/compressed CO2 continuous-flow system" ANGEWANDTE CHEMIE. INTERNATIONAL EDITION., Bd. 40, Nr. 14, 2001, Seiten 2697-2699, XP001071531 DEVERLAG CHEMIE. WEINHEIM.

## Beschreibung

Die Erfindung betrifft Alkylpyridiniumdicyanamide der Formel 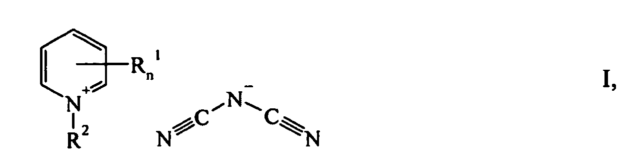 worin Rₙ¹ Cyano oder C₁₋₂₀-Alkyl bedeutet und n eine ganze Zahl von 0 bis 3 ist und die gegebenenfalls vorhandenen Reste R¹ gleich oder unterschiedlich sind, und worin R² C₁₋₂₀-Alkyl ist. Sie betrifft weiterhin deren Herstellung aus Alkylpyridiniumhalogeniden und Alkalidicyanamiden, sowie deren Verwendung als polare Lösungsmittel.

Alkylpyridiniumdicyanamide der Formel I sind ionische Flüssigkeiten. Unter ionischen Flüssigkeiten werden gemeinhin Salze verstanden, die organische Kationen enthalten und bei Raumtemperatur flüssig sind. Der Temperaturbereich, in dem ionische Flüssigkeiten als Lösungsmittel verwendet werden können, deckt Temperaturen von -60 °C bis über 300 °C ab. Sie sind durch ihre guten Solvatationseigenschaften und ihre geringe Flüchtigkeit in den letzten Jahren als umweltfreundliche Lösungsmittel für eine "grüne Chemie" bekannt geworden.

Üblicherweise sind die Kationen in ionischen Flüssigkeiten einwertige quartäre Ammonium- oder Phosphoniumbasen, oder Kationen von aromatischen stickstoffhaltigen Basen, welche gegebenenfalls mit Alkylgruppen, Halogenatomen oder Cyanogruppen substituiert sind und weitere Heteroatome wie O oder S enthalten können. Beispiele für stickstoffhaltige Kationen sind Imidazolium-, Oxazolium-, Pyrazinium-, Pyrazolium-, Pyridazinium-, Pyridinium-, Pyrrolidinium-, Pyrimidinium-, Thiazolium- und Triazoliumionen. Imidazolium- und Pyrrolidiniumionen werden am häufigsten verwendet. In Strukturformeln wird die Ladung lokalisiert auf dem Heteroatom (meist am Stickstoff) oder delokalisiert in der Mitte des Ringes dargestellt. Beide Darstellungsweisen sind äquivalent.

Typische Anionen in ionischen Flüssigkeiten sind Acetat, AlCl₄⁻, AsF₆⁻, BF₄⁻, Bromid, CF₃SO₃⁻, (CF₃)₂PF₄⁻, (CF₃)₃PF₃⁻, (CF₃)₄PF₂⁻, (CF₃)₅PF⁻, (CF₃)₆P⁻, Chlorid, CN⁻, FeCl₃⁻, NO₃⁻, PF₆⁻, Pyruvat, Trifluormethansulfonat, Oxalat oder SCN⁻. Am häufigsten werden AlCl₄⁻, AsF₆⁻, BF₄⁻ und PF₆⁻ verwendet.

Ausgangsmaterialien für die Herstellung von ionischen Flüssigkeiten können beispielsweise von Merck KGaA, Darmstadt oder IoLiTec, A. Bösmann, Dr. T. Schubert G.b.R., Freiburg bezogen werden.

Ionische Flüssigkeiten mit Pyridiniumionen und deren Verwendung sind unter anderem offenbart in US-A-2455331, WO-A1-02/34863, WO-A2-03/004727, US-A1-2004/0038031 und US-A1-2004/0031685.

Weitere bekannte Anwendungen von ionischen Flüssigkeiten auf Pyridiniumbasis sind offenbart in A. Ikeda et al. Chemistry Letters 2001, 1154-1155; M. Grätzel et al. J. Phys. Chem. B 107, 2003, 13280-13285; G.L. Rebeiro und B.M. Khadilkar Synthesis 3, 2001, 370-372; A.J. Carmichael und M.J. Earle Organic Letters 1, 1999, 997-1000; A. Boesmann et al. Angew. Chem. Int. Ed. 40, 2001, 2697-2699 und A. Eleuteri und D. Capaldi Org. Proc. Res. Dev. 4, 2000, 182-189.

Ionische Flüssigkeiten, die ein Dicyanamidion enthalten (*N,N*-Dialkylimidazolium, *N,N*-Dialkylpyrrolidinium- und Tetraalkylammoniumdicyanamid) sind offenbart in D.R. MacFarlane, et al. Chem. Commun. 2001, 1430-1431; S.A. Forsyth et al. Chem. Commun. 2002, 714-715 und D.R. MacFarlane, et al. Green Chemistry. 4, 2002, 444-448. Alle offenbarten *N,N*-Dialkylimidazolium, *N,N*-Dialkylpyrrolidinium- und Tetraalkylammoniumdicyanamide werden aus den entsprechenden Imidazolium-, Pyrrolidinium- und Tetraalkylammoniumiodiden mit Silberdicyanamid (Ag(C₂N₃)) hergestellt. Andere Herstellungsweisen sind nicht offenbart, ebensowenig Beispiele für die Herstellung der genannten Dicyanamide aus anderen Halogeniden.

Aufgabe der vorliegenden Erfindung war es, neue ionische Flüssigkeiten und kostengünstige Verfahren zu deren Herstellung zur Verfügung zu stellen. Die neuen Verbindungen sollten nach Gebrauch umweltschonend entsorgt werden können.

Diese Aufgabe wird entsprechend Anspruch 1 gelöst.

Beansprucht werden Alkylpyridiniumdicyanamide der Formel 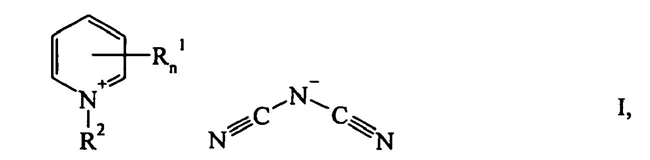 worin Rₙ¹ Cyano oder C₁₋₂₀-Alkyl bedeutet und n eine ganze Zahl von 0 bis 3 ist und die gegebenenfalls vorhandenen Reste R¹ gleich oder unterschiedlich sind, und worin R² C₁₋₂₀-Alkyl ist.

Hier und im Folgenden bedeutet der Ausdruck "C₁₋ₙ-Alkyl" eine unverzweigte oder verzweigte Alkylgruppe mit 1 bis n Kohlenstoffatomen. C₁₋₂₀-Alkyl repräsentiert beispielsweise Methyl, Ethyl, Propyl, Isopropyl, Butyl, Isobutyl, *sec*-Butyl, *tert*-Butyl, Pentyl, 1,4-Dimethyl-pentyl, Hexyl, Heptyl, Octyl, 1,5-Dimethyl-hexyl, Nonyl, Decyl und 4-Ethyl-1,5-dimethylhexyl, Undecyl, Dodecyl, Tridecyl, Tetradecyl oder Eicosyl.

Bevorzugte Alkylpyridiniumdicyanamide sind Verbindungen der Formel I, wobei Rₙ¹ Cyano oder C₁₋₈-Alkyl bedeutet, besonders bevorzugt Methyl und Ethyl, und wobei n eine ganze Zahl von 0 bis 2 ist und die gegebenenfalls vorhandenen Reste R¹ gleich oder unterschiedlich sind, und worin R² C₂₋₈-Alkyl ist. Besonders bevorzugt sind Alkylpyridiniumdicyanamide entsprechend der Formel 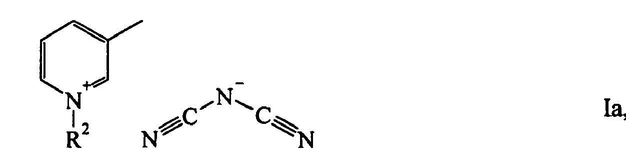 worin R² C₄₋₈-Alkyl ist.

Die in ionischen Flüssigkeiten üblicherweise verwendeten Anionen haben den Nachteil, dass bei ihrer thermischen Entsorgung oftmals halogen- und metallhaltige Rückstände entstehen. Bei der thermischen Entsorgung der erfindungsgemässen Alkylpyridiniumdicyanamide entstehen diese Abfälle nicht.

Durch das aromatische Gerüst eröffnen die erfindungsgemässen ionischen Flüssigkeiten mit gegebenenfalls substituierten pyridinbasierten Kationen einen breiteren Polaritätsbereich als beispielsweise solche aus quartären Ammoniumionen.

Die Wasserlöslichkeit der erfindungsgemässen Alkylpyridiniumdicyanamide lässt sich durch die Zahl und Kettenlänge der Substituenten Rₙ¹ und R² in den erfindungsgemässen Verbindungen in einer Bandbreite von "vollständig mit Wasser mischbar" bis hin zu "mit Wasser unmischbar" einstellen. So sind beispielsweise *N*-Butyl-pyridinium- und *N*-Butyl-3-methylpyridiniumdicyanamid mit Wasser mischbar, während *N*-Octyl-3-methyl-pyridinium- und *N*-Octyl-pyridiniumdicyanamid nicht mit Wasser mischbar sind. Die Mischbarkeit mit Lösungsmitteln wie beispielsweise Aceton, Acetonitril, DMSO, Ethylacetat, Hexan, Methylenchlorid, organischen Säuren, Propylencarbonat, Schwefelkohlenstoff, THF, Toluol oder anderen ionischen Flüssigkeiten wird ebenfalls von den Seitengruppen Rₙ¹ und R² wesentlich mitbestimmt.

Ein Verfahren zur Herstellung der erfindungsgemässen Alkylpyridiniumdicyanamide der Formel 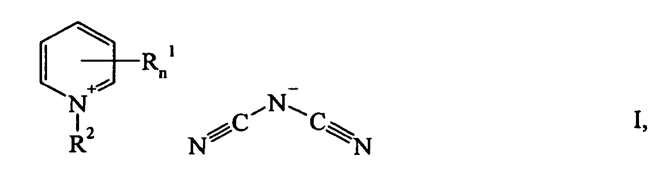 worin Rₙ¹ Cyano oder C₁₋₁₀-Alkyl bedeutet und n eine ganze Zahl von 0 bis 3 ist und die gegebenenfalls vorhandenen Reste R¹ gleich oder unterschiedlich sind, und worin R² C₁₋₁₀-Alkyl ist, umfasst die Umsetzung einer Verbindung der Formel 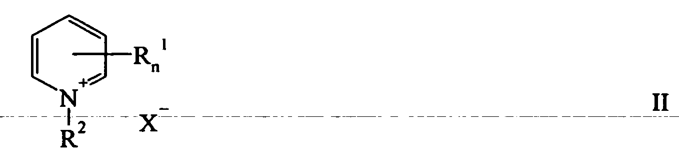 worin Rₙ¹ und R² wie oben definiert sind und X ein Halogenatom ausgesucht aus der Gruppe bestehend aus Fluor, Chlor, Brom und Iod ist, gegebenenfalls in Gegenwart von Wasser, mit einem Alkalidicyanamid.

Unter Alkalidicyanamiden werden hier und im Folgenden Dicyanamide der Alkalimetalle Lithium, Natrium, Kalium, sowie deren Mischungen und Hydrate verstanden.
Die Alkalidicyanamide können als Feststoff, in Lösung oder Suspension eingesetzt werden, besonders bevorzugt werden sie als wässrige Lösung oder Suspension eingesetzt.

In einer bevorzugten Ausführungform ist das molare Verhältnis der Reaktanden Alkylpyridiniumhalogenid : Alkalidicyanamid in einem Bereich von 0,2 : 1 bis 5 : 1,0, besonders bevorzugt im Verhältnis von 1 : 1. Nicht umgesetzte Ausgangsprodukte lassen sich einfach vom entstehenden Alyklpyridiniuimdicyanamid abtrennen.

In einer bevorzugten Verfahrensvariante wird die Umsetzung von Alkylpyridiniumhalogenid und Alkalidicyanamid in Gegenwart von Wasser in einem molaren Verhältnis der Summe der Reaktanden zu Wasser von 2 : 0 bis 2 : 100, vorzugsweise von 2 : 10 bis 2 : 30, besonders bevorzugt von 2 : 15 bis 2 : 25 ausgeführt.

Das erfindungsgemässe Verfahren zeichnet sich gegenüber dem bekannten Verfahren zur Herstellung von ionischen Flüssigkeiten mit Dicyanamidionen dadurch aus, dass auf die Herstellung des Ag(C₂N₃) als Zwischenverbindung verzichtet werden kann.

Durch die hohe Konzentration der Ausgangsverbindungen im erfindungsgemässen Verfahren lassen sich Alkylpyridiniumdicyanamide überraschend einfach und kostengünstig direkt aus Alkylpyridiniumhalogeniden und Alkalidicyanamiden herstellen. Damit entfällt auch die kostspielige Aufarbeitung der anfallenden silberhaltigen Abfälle. Weiterhin können im erfindungsgemässen Verfahren nicht nur Iodide, sondern ohne Ausbeuteverluste auch die weitaus günstigeren anderen Halogenide eingesetzt werden.
In einer weiteren bevorzugten Verfahrensvariante werden abhängig von Anzahl und Eigenschaften der Reste Rₙ¹ beziehungsweise R² neben Wasser noch weitere Lösungsmittel wie beispielsweise Aceton, Acetonitril, C₁₋₄-Alkohole, Chloroform, Dichlorethan, Diethylether, DMSO, Ethylacetat, Hexan, Methylenchlorid, Propylencarbonat, Schwefelkohlenstoff, THF, Toluol und/oder Xylol als Lösungsvermittler eingesetzt.

In einer bevorzugten Ausführungsform werden die Alkylpyridiniumdicyanamide durch ein Extraktionsverfahren gereinigt. Besonders bevorzugt können Alkylpyridindicyanamide und entstehende Alkalihalogenide in Gegenwart von Wasser durch Phasentrennung isoliert werden. Wenn in der Reaktionslösung bereits Wasser vorhanden ist, bilden auch ansonsten vollständig mit Wasser mischbare Alkylpyridiniumdicyanamide durch während der Reaktion entstehendes Alkalihalogenid eine Phasengrenze aus (Aussalzeffekt). Dieser Aussalzeffekt kann durch Salzzugabe, beispielsweise während der Extraktion, verstärkt werden.

Weiterhin konnte überraschend festgestellt werden, dass sich selbst gut wasserlösliche Alkylpyridiniumdicyanamide mit nicht wassermischbaren organischen Lösungsmitteln niedriger Polarität, wie beispielsweise Methylenchlorid, aus wässrigen Lösungen extrahieren lassen.

Pyridin und verschiedene Alkylpyridine kommen in natürlichen Quellen, wie beispielsweise im Steinkohlenteer vor und werden dort technisch einfach und in grossen Mengen isoliert. Da Imidazole nicht natürlich vorkommen, haben die erfindungsgemässen Alkylpyridiniumdicyanamide einen Kostenvorteil gegenüber bekannten Imidazoliumdicyanamiden. Substituierte Pyridine lassen sich nach einer Vielzahl von Methoden herstellen. Die *N*-Alkylierung der Pyridine erfolgt ebenfalls nach bekannten Verfahren, beispielsweise mit Alkylhalogeniden. Da Pyridine nur ein alkylierbares Stickstoffatom im Ring haben, entstehen gerade bei der *N*-Alkylierung einheitliche Produkte, während die *N*-Alkylierung von substituierten Imidazolderivaten oft zu uneinheitlichen, aber schwer zu trennenden Produkten führen kann.

Die erfindungsgemässen Alkylpyridiniumdicyanamide der Formel 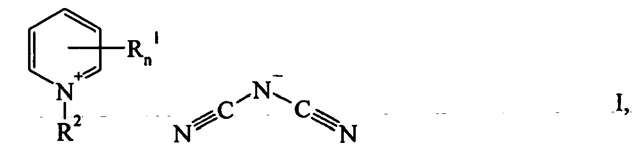 worin Rₙ¹ Cyano oder C₁₋₁₀-Alkyl bedeutet und n eine ganze Zahl von 0 bis 3 ist und die gegebenenfalls vorhandenen Reste R¹ gleich oder unterschiedlich sind, und worin R² C₁₋₁₀-Alkyl ist, sind ionische Flüssigkeiten und können, gegebenenfalls in einer Mischung mit einer oder mehreren anderen ionischen Flüssigkeiten, Wasser oder organischen Lösungsmitteln, verwendet werden. Verwendungsmöglichkeiten der erfindungsgemässen Alkylpyridiniumdicyanamide sind beispielsweise als Bestandteil von polaren Lösungsmitteln, als Elektrolyte in der Elektrolyse oder in elektrischen Bauteilen oder für die Herstellung von Flüssigkristallen für LCD's oder von Leitgelen, beispielsweise für Anwendungen in der Photovoltaik.

Weiterhin wurde gefunden, dass bei Suzuki-Reaktionen (C-C-Verknüpfung von Halogenaromaten mit aromatischen Borsäure-Estern) in den erfindungsgemässen ionischen Flüssigkeiten weniger Nebenprodukte generiert werden, als bei der Verwendung von ionischen Flüssigkeiten auf Basis der bekannten Imidazoliumdicyanamide.

Die folgende Beispiele sollen die Erfindung verdeutlichen, ohne jedoch eine Einschränkung darzustellen.

### Beispiele:

### Beispiel 1:

### 3-Methyl-1-butyl-pyridiniumchlorid (Formel II, R¹ = 3-Methyl, R² = Butyl, X = Cl)

3-Methylpyridin (2142 g, 23 mol) und 1-Chlorbutan (2129 g, 23 mol) werden in einem 5 L Doppelmantelrührwerk vorgelegt und unter Rückfluss gerührt. Die Temperatur im Rührwerk beträgt ca. 92-95 °C. Aus der anfänglich klaren Lösung bilden sich zunächst 2 Phasen, von denen die untere das Produkt enthält. Nach vollständigem Umsatz liegt nur noch eine Phase vor. Das Produkt kann batchweise oder continuierlich durch Phasentrennung einfach von den Ausgangsmaterialien getrennt werden. Nach 33 h werden 1042 g rohes 3-Methyl-1-butyl-pyridiniumchlorid erhalten. Reste von 3-Methylpyridin und 1-Chlorbutan (<5%) werden durch Vakuumbehandlung bei ca. 90 °C entfernt. Die Reinheit entspricht gemäss ¹H-NMR >98%.

### Beispiel 2:

### 3-Methyl-1-butyl-pyridiniumdicyanamid (Formel I, R¹ = 3-Methyl, R² = Butyl)

Eine wässrige Lösung aus 3-Methyl-1-butyl-pyridiniumchlorid (1686 g, 9 mol) in Wasser (2550 mL, 140 mol) wird bei Raumtemperatur portionsweise mit festem Natriumdicyanamid (808 g, 9 mol) versetzt und anschliessend geführt bis sich alle Feststoffe lösen. Dabei bildet sich eine klare beigefarbene Lösung. Die Lösung wird mit 85 g Aktivkohle versetzt und für 30 min gerührt und anschliessend filtriert. Die klare gelbliche Lösung wird dann mit je Dichlormethan (2 × 1000 mL) extrahiert. Die Extrakte werden dann mit Wasser (3 × 500 mL) gewaschen und dann zunächst bei 50 °C und 500 mbar, später 20 mbar bis zur Gewichtskonstanz eingeengt. Man erhält auf diese Weise 1708 g 3-Methyl-1-butyl-pyridiniumdicyanamid in Form einer einer hellbeigen Flüssigkeit, entsprechend 87 % Ausbeute und mit einer Reinheit von >98 % gemäss ¹H-NMR.

### Beispiel 3:

### 3-Methyl-1-octyl-pyridiniumchlorid (Formel II, R¹ = 3-Methyl, R² = Octyl, X = Cl)

3-Methylpyridin (1676 g, 18 mol) und 1-Chloroctan (2676 g, 18 mol) werden in einem 5 L Doppelmantelrührwerk vorgelegt und bei einer Temperatur von 130-135 °C gerührt. Aus der anfänglich klaren Lösung bilden sich 2 Phasen, von denen die untere das 3-Methyl-1-octyl-pyridiniumchlorid enthält. Im Verlauf der Reaktion nimmt die obere Phase immer weiter ab, bis nach vollständigen Umsatz das Reaktionsgemisch wiederum nur aus einer Phase besteht. Nach 24 h werden 4370 g Rohprodukt erhalten. Das erhaltene beige bis bräunliche Reaktionsprodukt erstarrt langsam beim Abkühlen auf Raumtemperatur. Reinheit gemäss ¹H-NMR >98%, Schmelzbereich 63-70 °C.

### Beispiel 4:

### 3-Methyl-1-octyl-pyridiniumdicyanamid (Formel I, R¹ = 3-Methyl, R² = Octyl)

Eine Mischung aus 3-Methyl-1-octyl-pyridiniumchlorid (242 g, 1 mol) und wenig Wasser (25 mL, 1.4 mol) wird bei Raumtemperatur innerhalb von 30 min mit einer gesättigten Lösung von Natriumdicyanamid (89 g, 1 mol) in Wasser (340 mL, 18.9 mol) versetzt. Nach vollständiger Zudosierung wird die Reaktionsmischung noch für weitere 20 min gerührt. Dabei bilden sich zwei Phasen. Die organische Phase (unten) wird abgetrennt und die wässrige Phase mit Dichlormethan (2 × 300 mL) extrahiert. Die Dichlormethanextrakte und die zuvor abgetrennte organische Phase werden vereinigt und mit Wasser (3 × 250 mL) gewaschen. Die organische Phase wird zunächst bei zuerst bei 500 mbar und 50 °C, später bei 20 mbar und 50 °C bis zur Gewichtskonstanz eingeengt. Man erhält auf diese Weise 218 g einer hellbeigen klaren Flüssigkeit (entsprechend 90% Ausbeute) an 3-Methyl-1-octyl-pyridiniumdicyanamid mit einer Reinheit von >98% gemäss ¹H-NMR.

## Patentansprüche

1. Alkylpyridiniumdicyanamide der Formel 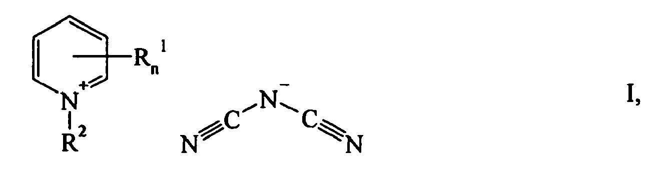 worin Rₙ¹ Cyano oder C₁₋₂₀-Alkyl bedeutet und n eine ganze Zahl von 0 bis 3 ist und die gegebenenfalls vorhandenen Reste R¹ gleich oder unterschiedlich sind, und worin R² C₁₋₂₀-Alkyl ist.

2. Alkylpyridiniumdicyanamide nach Anspruch 1, worin Rₙ¹ Cyano oder C₁₋₈-Alkyl bedeutet, vorzugsweise Methyl oder Ethyl, und n eine ganze Zahl von 0 bis 2 ist und die gegebenenfalls vorhandenen Reste R¹ gleich oder unterschiedlich sind, und worin R² C₂₋₈-Alkyl ist.

3. Alkylpyridiniumdicyanamide nach Anspruch 1 der Formel 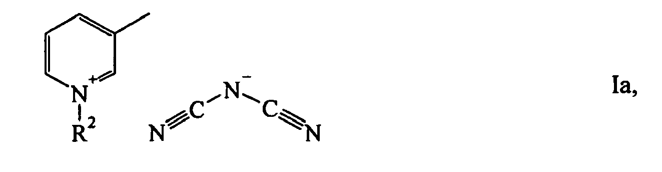 **dadurch gekennzeichnet, dass** R² C₄₋₈-Alkyl ist.

4. Verfahren zur Herstellung von Alkylpyridiniumdicyanamiden gemäss Anspruch 1, **dadurch gekennzeichnet, dass** ein Alkylpyridiniumhalogenid der Formel 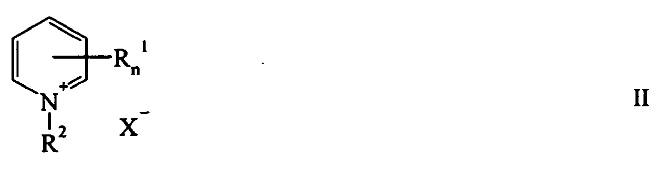 worin Rₙ¹ Cyano oder C₁₋₂₀-Alkyl bedeutet und n eine ganze Zahl von 0 bis 3 ist und die gegebenenfalls vorhandenen Reste R¹ gleich oder unterschiedlich sind, und worin R² C₁₋₂₀-Alkyl ist, und worin X⁻ ein Halogenion ist, vorzugsweise in Gegenwart von Wasser, mit einem Alkalidicyanamid umgesetzt wird.

5. Verfahren nach Anspruch 4, **dadurch gekennzeichnet, dass** X⁻ Chlorid oder Bromid ist.

6. Verfahren nach Anspruch 4 oder 5, **dadurch gekennzeichnet, dass** das molare Verhältnis von Alkylpyridiniumhalogenid : Alkalidicyanamid in einem Bereich von 0,2 : 1 bis 5 : 1, besonders bevorzugt im Verhältnis von 1 : 1 ist.

7. Verfahren nach mindestens einem der Ansprüche 4 bis 6, **dadurch gekennzeichnet, dass** die Umsetzung von Alkylpyridiniumhalogenid und Alkalidicyanamid in Gegenwart von Wasser in einem molaren Verhältnis der Summe der Reaktanden zu Wasser von 2 : 0 bis 2 : 100, vorzugsweise von 2 : 10 bis 2 : 30, besonders bevorzugt von 2 : 15 bis 2 : 25 ausgeführt wird.

8. Verfahren nach mindestens einem der Ansprüche 4 bis 7, **dadurch gekennzeichnet, dass** das Alkalidicyanamid als Lösung oder Suspension eingesetzt wird.

9. Verwendung von Alkylpyridiniumdicyanamiden gemäss Anspruch 1, gegebenenfalls in einer Mischung mit einer oder mehreren anderen ionischen Flüssigkeiten, Wasser oder organischen Lösungsmitteln, als polare aprotische Lösungsmittel, als Elektrolyte in der Elektrolyse oder in elektrischen Bauteilen und für die Herstellung von Flüssigkristallen für LCD's oder von Leitgelen.

10. Verwendung von Alkylpyridiniumdicyanamiden gemäss Anspruch 1, gegebenenfalls in einer Mischung mit einer oder mehreren anderen ionischen Flüssigkeiten, Wasser oder organischen Lösungsmitteln, als Lösungsmittel in Suzuki-Reaktionen.

## Claims

1. Alkylpyridinium dicyanamides of the formula 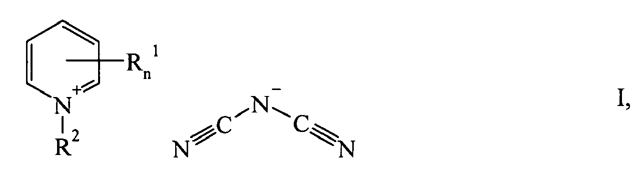 in which Rₙ¹ is cyano or C₁₋₂₀-alkyl and n is an integer from 0 to 3 and any R¹ radicals present are the same or different, and in which R² is C₁₋₂₀-alkyl.

2. Alkylpyridinium dicyanamides according to Claim 1, in which Rₙ¹ is cyano or C₁₋₈-alkyl, preferably methyl or ethyl, and n is an integer from 0 to 2 and any R¹ radicals present are the same or different, and in which R² is C₂₋₈-alkyl.

3. Alkylpyridinium dicyanamides according to Claim 1 of the formula 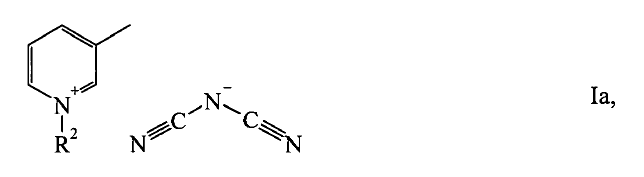 **characterized in that** R² is C₄₋₈-alkyl.

4. Process for preparing alkylpyridinium dicyanamides according to Claim 1, **characterized in that** an alkylpyridinium halide of the formula 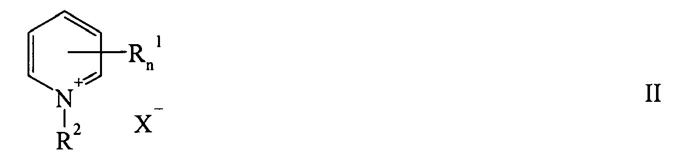 where Rₙ¹ is cyano or C₁₋₂₀-alkyl and n is an integer from 0 to 3 and any R¹ radicals present are the same or different, and in which R² is C₁₋₂₀-alkyl, and in which X⁻ is a halogen ion, is reacted with an alkali metal dicyanamide, preferably in the presence of water.

5. Process according to Claim 4, **characterized in that** X⁻ is chloride or bromide.

6. Process according to Claim 4 or 5, **characterized in that** the molar ratio of alkylpyridinium halide: alkali metal dicyanamide is within a range of 0.2 : 1 to 5 : 1, more preferably in the ratio of 1 : 1.

7. Process according to at least one of Claims 4 to 6, **characterized in that** the reaction of alkylpyridinium halide and alkali metal dicyanamide is performed in the presence of water in a molar ratio of the sum of the reactants to water of 2 : 0 to 2 : 100, preferably of 2 : 10 to 2 : 30, more preferably of 2 : 15 to 2 : 25.

8. Process according to at least one of Claims 4 to 7, **characterized in that** the alkali metal dicyanamide is used in the form of a solution or suspension.

9. Use of alkylpyridinium dicyanamides according to Claim 1, if appropriate in a mixture with one or more other ionic liquids, water or organic solvents, as a polar aprotic solvent, as an electrolyte in electrolysis or in electrical components, and for the production of liquid crystals for LCDs or of conductive gels.

10. Use of alkylpyridinium dicyanamides according to Claim 1, if appropriate in a mixture with one or more other ionic liquids, water or organic solvents, as a solvent in Suzuki reactions.

## Revendications

1. Dicyanamides d'alkylpyridinium de formule 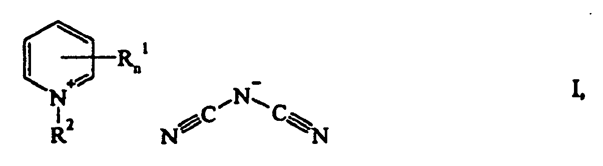 dans laquelle Rₙ¹ signifie cyano ou alkyle en C₁₋₂₀ et n est un nombre entier de 0 à 3, et les radicaux R¹ éventuellement présents sont identiques ou différents, et dans laquelle R² est un alkyle en C₁₋₂₀.

2. Dicyanamides d'alkylpyridinium selon la revendication 1, dans lesquels Rₙ¹ signifie cyano ou alkyle en C₁₋₈, de préférence méthyle ou éthyle, et n est un nombre entier de 0 à 2, et les radicaux R¹ éventuellement présents sont identiques ou différents, et dans lesquels R² est un alkyle en C₂₋₈.

3. Dicyanamides d'alkylpyridinium selon la revendication 1, de formule 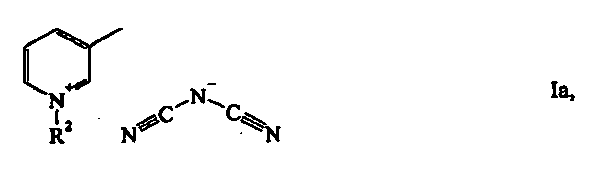 **caractérisés en ce que** R² est un alkyle en C₄₋₈.

4. Procédé de fabrication de dicyanamides d'alkylpyridinium selon la revendication 1, **caractérisé en ce qu'**un halogénure d'alkylpyridinium de formule 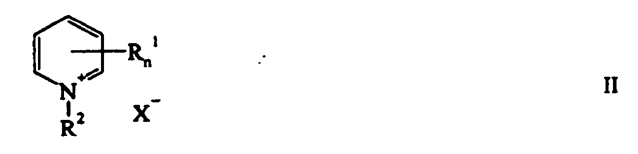 dans laquelle Rₙ¹ signifie cyano ou alkyle en C₁₋₂₀ et n est un nombre entier de 0 à 3, et les radicaux R¹ éventuellement présents sont identiques ou différents, et dans laquelle R² est un alkyle en C₁₋₂₀, et dans laquelle X- est un ion halogène, est mis en réaction, de préférence en présence d'eau, avec un dicyanamide alcalin.

5. Procédé selon la revendication 4, **caractérisé en ce que** X- est un chlorure ou un bromure.

6. Procédé selon la revendication 4 ou 5, **caractérisé en ce que** le rapport molaire halogénure d'alkylpyridinium:dicyanamide alcalin est dans la plage allant de 0,2:1 à 5:1, de manière particulièrement préférée de 1:1.

7. Procédé selon au moins l'une quelconque des revendications 4 à 6, **caractérisé en ce que** la réaction de l'halogénure d'alkylpyridinium et du dicyanamide alcalin est réalisée en présence d'eau en un rapport molaire entre la somme des réactifs et l'eau de 2:0 à 2:100, de préférence de 2:10 à 2:30, de manière particulièrement préférée de 2:15 à 2:25.

8. Procédé selon au moins l'une quelconque des revendications 4 à 7, **caractérisé en ce que** le dicyanamide alcalin est utilisé sous la forme d'une solution ou d'une suspension.

9. Utilisation de dicyanamides d'alkylpyridinium selon la revendication 1, éventuellement en mélange avec un ou plusieurs autres liquides ioniques, de l'eau ou des solvants organiques, en tant que solvants aprotiques polaires, en tant qu'électrolytes dans l'électrolyse ou dans des composants électriques et pour la fabrication de cristaux liquides pour LCD ou de gels conducteurs.

10. Utilisation de dicyanamides d'alkylpyridinium selon la revendication 1, éventuellement en mélange avec un ou plusieurs autres liquides ioniques, de l'eau ou des solvants organiques, en tant que solvants dans des réactions de Suzuki.
